# EUROPÄISCHE PATENTSCHRIFT

(11) **EP 1 267 980 B1**
(45) Veröffentlichungstag und Bekanntmachung des Hinweises auf die Patenterteilung: **26.03.2008**
(21) Anmeldenummer: 02700193.2
(22) Anmeldetag: 05.01.2002
(51) Int. Cl.: A61M 19/00, A61M 25/00

(54) **KATHETER FÜR DIE NERVENBLOCKADE**
CATHETER FOR NEURAL BLOCKADES
CATHETER POUR BLOCS NERVEUX

(30) Priorität: 11.01.2001 DE 10100976
(43) Veröffentlichungstag der Anmeldung: 02.01.2003
(73) Patentinhaber: Pajunk, Heinrich, 78187 Geisingen (DE); Pajunk, Horst, 78187 Geisingen (DE)
(72) Erfinder: Pajunk, Heinrich, 78187 Geisingen (DE); Pajunk, Horst, 78187 Geisingen (DE)
(74) Vertreter: Patentanwälte Westphal, Mussgnug & Partner
(86) Internationale Anmeldenummer: PCT/EP2002/000049
(87) Internationale Veröffentlichungsnummer: WO 2002/055145

(56) Entgegenhaltungen:
- EP-A- 1 002 500
- AT-B- 406 121
- DE-A- 19 807 487

## Beschreibung

Die Erfindung betrifft einen Katheter für die Nervenblockade in der Anästhesie gemäß dem Oberbegriff des Anspruchs 1.

In der Anästhesie werden für länger dauernde operative Eingriffe, für postoperative Schmerztherapie und für die Behandlung chronischer Schmerzzustände, die einen gewissen Körperbereich versorgenden Nerven durch ein Einleiten eines Anästhetikums blockiert. Zum Einleiten des Anästhetikums wird ein Katheter benutzt, dessen distales Ende möglichst nahe an dem Nerv positioniert wird, um mit einer möglichst geringen Menge des Anästhetikums eine optimale Wirkung zu erzielen. Der Katheter, der im Bedarfsfalle auch über eine längere Zeit in situ verbleiben kann, besteht aus einem langen dünnen flexiblen Kunststoffschlauch.

Um den Katheter in die Nervenscheide oder den Nervenkanal einzuführen, wird eine Kanüle verwendet, durch welche der Katheter eingeführt wird. Aus der US 5007902 ist es bekannt, eine solche Kanüle mittels einer Stichkanüle zu setzen, wobei die Stichkanüle aus der Kanüle herausgezogen wird, um den Katheter einführen zu können. Aus der DE 3643235 C1 ist eine Stichkanüle bekannt, deren Innenkanal seitlich hinter der distalen Kanülenspitze austritt, wobei der Katheter durch diese Stichkanüle eingeführt und plaziert wird. Nachdem der Katheter plaziert ist, kann die zu seinem Einführen dienende Kanüle entfernt werden.

Aus der US 5007902 ist es bekannt, zur genau lokalisierten Plazierung des Katheters die Elektrostimulation des Nerven auszunützen. Hierzu wird in dem Katheter ein Draht als Mandrin eingesetzt, dessen distales Ende geringfügig aus dem distalen Ende des Katheters herausragt. Das proximale Ende des Mandrins ist für den Anschluß eines Stimulators elektrisch kontaktierbar. Durch die Elektrostimulation kann die Lage des distalen Endes des Katheters exakt bestimmt werden, während der Katheter in die Nervenscheide oder den Nervenkanal eingeführt wird. Da der Draht als Mandrin den Innenquerschnitt des Katheters vollständig ausfüllt, muß der Draht aus dem Katheter herausgezogen werden, sobald der Katheter plaziert ist, um das Anästhetikum durch den Katheter applizieren zu können. Wenn der Katheter über eine längere Zeitdauer verbleibt und erneut ein Medikament eingeleitet werden muß, ist es häufig erforderlich, die Lage des Katheters zu prüfen und ggf. zu korrigieren. Hierzu ist es notwendig, erneut einen Drahtmandrin einzuführen, um die Lage des distalen Endes des Katheters durch Elektrostimulation zu bestimmen.

Weiter ist es bekannt, in dem Katheter einen dünnen Draht für die Elektrostimulation anzuordnen, dessen Querschnitt wesentlich geringer ist als der freie Innenquerschnitt des Katheters. Der Draht behindert somit nicht das Einleiten einer Flüssigkeit durch den Katheter, so dass der Draht in dem Katheter verbleiben kann. Über den fest in dem Katheter angeordneten Draht kann während der gesamten Verweildauer des Katheters dessen Lage durch Elektrostimulation kontrolliert und korrigiert werden. Ragt der dünne Draht aus dem distalen Ende des Katheters heraus, so besteht allerdings ein Risiko, dass die Drahtspitze beim Vorschieben des Katheters Schädigungen und Verletzungen des Nerven verursacht. Ragt der Draht nicht aus dem distalen Ende des Katheters heraus, so wird zwar dieses Risiko verringert, es ist jedoch kein zuverlässiger elektrischer Kontakt für die Elektrostimulation mehr gewährleistet.

Der Erfindung liegt die Aufgabe zugrunde, einen Katheter für die Nervenblockade zur Verfügung zu stellen, der mittels Elektrostimulation plaziert werden kann, wobei ein zuverlässiger Kontakt für die Elektrostimulation und ein minimales Verletzungsrisiko gewährleistet sind und eine Kontrolle der Lage des Katheters durch Elektrostimulätion jederzeit möglich ist.

Diese Aufgabe wird erfingdungsgemäß gelöst durch einen Katheter mit den Merkmalen des Anspruchs 1.

Vorteilhafte Ausführungen und Weiterbildungen der Erfindung sind in den Unteransprüchen angegeben.

Der wesentliche Gedanke der Erfindung besteht darin, in das distale Ende des Katheters eine Kontaktspitze einzusetzen, die aus einem Metallteil besteht, welches mit einem Stopfen koaxial in das distale Ende des Katheters eingesetzt ist und mit einer Kappe vor das distale Ende des Katheters ragt und dessen Stirnrandfläche übergreift. Zur elektrisch leitenden Verbindung dieser Kontaktspitze mit dem proximalen Ende ist in dem Katheter ein dünner Draht angeordnet, der elektrisch leitend mit dem Stopfen der Kontaktspitze verbunden ist. Die Kontaktspitze bildet mit ihrer Kappe das distale Ende des Katheters. Die Kappe gewährleistet einen zuverlässigen großflächigen elektrischen Kontakt für die Nervenstimulation. Die stumpf vorgewölbte und den distalen Rand des Katheters übergreifende Kappe verhindert außerdem das Risiko von Nervenverletzungen beim Vorwärtsschieben des Katheters. Der Stopfen, der in das distale Ende des Katheters eingesetzt ist, hält die Kontaktspitze zentriert in dem distalen Ende des Katheters. Der dünne Draht, der in dem Katheter verläuft, um die distale Kontaktspitze mit dem proximalen Anschluß des Stimulators zu verbinden, behindert das Einleiten von Flüssigkeit, z.B. eines Anästhetikums nicht. Eine Elektrostimulation ist beim Einführen des Katheters zur exakten Plazierung möglich und kann auch jederzeit bei liegendem Katheter wiederholt werden, um dessen Lage zu kontrollieren und ggf. zu korrigieren.

Die Kontaktspitze kann das distale Ende des Katheters vollständig verschließen. In diesem Falle tritt die über den Katheter eingeleitete Flüssigkeit distal über Austrittsöffnungen aus, die in der Wandung des Katheters in dessen distalem Endbereich unmittelbar hinter der Kontaktspitze angeordnet sind. In einer anderen Ausführung kann die Kontaktspitze eine axiale Bohrung aufweisen, durch welche die Flüssigkeit austreten kann. In diesem Falle sind Austrittsöffnungen in der Wandung des Katheters nicht notwendig, können jedoch ggf. auch vorgesehen sein, um den Austrittsquerschnitt zu vergrößern. Die Austrittsöffnungen in der Wandung des Katheters sind vorzugsweise so ausgebildet, dass ihre Mittelachse, d.h. die Austrittsrichtung einen spitzen Winkel von weniger als 90° mit der Mittelachse des Katheters einschließt, wobei sich dieser spitze Winkel in distaler Richtung öffnet. Der spitze Winkel kann in einer bevorzugten Ausführung etwa 45° betragen. Die Ausbildung der Austrittsöffnungen unter einem nach vorn gerichteten Winkel bewirkt, dass die über den Katheter eingeleitete Flüssigkeit gezielt in distale Richtung austritt, so dass eine genauer lokalisierte Applikation der Flüssigkeit, z.B. eines Anästhetikums, möglich ist.

Im folgenden wird die Erfindung anhand von in der Zeichnung dargestellten Ausführungsbeispielen näher erläutert. Es zeigen
- Figur 1 im Axialschnitt das distale Ende des Katheters in einer ersten Ausführung,
- Figur 2 eine entsprechende Darstellung einer zweiten Ausführung,
- Figur 3 eine entsprechende Darstellung einer dritten Ausführung,
- Figur 4 eine entsprechende Darstellung einer vierten Ausführung,
- Figur 5 eine entsprechende Darstellung einer fünften Ausführung und
- Figur 6 eine teilweise axialgeschnittene Seitenansicht des distalen Endes des Katheters ohne Kontaktspitze.

Der Katheter 10 ist ein flexibler Schlauch aus Kunststoff, z.B. aus Polyamid. Die Länge des Katheters 10 hängt von seinem Verwendungszweck ab. Für die periphere Plexusanästhesie werden Katheter 10 mit einer Länge von etwa 400 mm verwendet, während bei der Peridural- und Spinalanästhesie längere Katheter 10 von z.B. 900 mm verwendet werden. Ebenso hängt der Durchmesser des Katheters 10 von der Verwendung ab. Ein Katheter mit 19 G weist beispielsweise einen Außendurchmesser von 1 mm und einen Innendurchmesser von 0,5 mm auf. Ein Katheter mit 20 G weist einen Außendurchmesser von 0,8 mm und einen Innendurchmesser von 0,4 mm auf. Ggf. werden auch dünnere Katheter 10 mit entsprechend kleineren Durchmessern verwendet.

In das distale Ende des Katheters 10 wird eine Kontaktspitze eingesetzt, die als Metallteil ausgebildet ist. Die Kontaktspitze besteht einstückig aus einem Stopfen 12 und einer Kappe 14. Der Stopfen 12 hat die Form eines Kreiszylinders, dessen Außendurchmesser mit dem Innendurchmesser des Katheters 10 übereinstimmt. Die Kontaktspitze kann auf diese Weise mit ihrem Stopfen 12 in das distale Ende des Katheters 12 eingesetzt werden, wobei der Stopfen 12 in den Katheter 10 im Pass-Sitz gehalten wird. Die Kappe 14 weist einen Außendurchmesser auf, der mit dem Außendurchmesser des Katheters 10 übereinstimmt. Beim Einsetzen der Kontaktspitze in das distale Ende des Katheters 10 wird der Stopfen 12 in das Lumen des Katheters 10 eingeschoben, bis die Kappe 14 an der distalen Stirnfläche des Katheters 10 anschlägt. Da der Durchmesser des Stopfens 12 mit dem Innendurchmesser des Katheters 10 übereinstimmt, hält der Stopfen 12 die Kontaktspitze zentriert an dem distalen Ende des Katheters 10, wobei die Kappe 14 die Randstirnfläche des Katheters 10 übergreift und abdeckt. Am Außenumfang schließt die Kappe 14 bündig mit dem Katheter 10 ab, so dass die Kappe 14 stufenlos in den Außenumfang des Katheters 10 übergeht.

Zur elektrisch leitenden Verbindung der Kontaktspitze mit einem am proximalen Ende des Katheters angeschlossenen Stimulator ist in dem Katheter 10 ein dünner Draht 16 angeordnet. Der Durchmesser des Drahtes 16 ist wesentlich kleiner als der Innendurchmesser des Katheters 10, so dass ein wesentlicher Teil des Innenlumens des Katheters 10 für das Durchleiten einer Flüssigkeit frei bleibt. Der Durchmesser des Drahtes 16 beträgt beispielsweise 0,2 mm. Das proximale Ende des Drahtes 16 ist in einer nicht dargestellten geeigneten Weise mit dem Stimulator kontaktierbar. Das distale Ende des Drahtes 16 ist mit dem Stopfen 12 der Kontaktspitze elektrisch leitend verbunden, z.B. weichgelötet, geklebt, geprägt oder lasergeschweißt.

Nachfolgend werden unterschiedliche Ausführungsbeispiele der Kontaktspitze beschrieben.

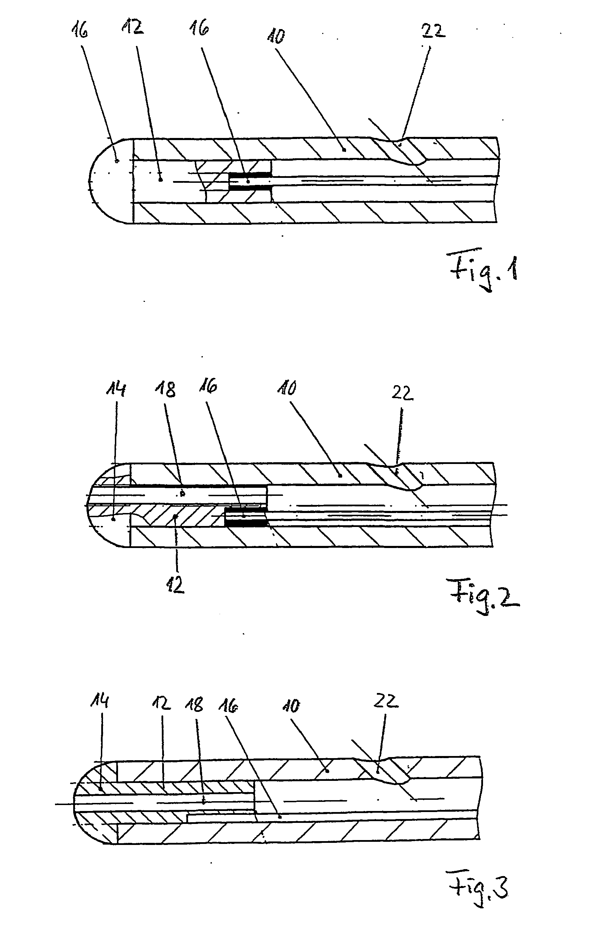

In dem Ausführungsbeispiel der Figur 1 sind der Stopfen 12 und die Kappe 14 geschlossen. Die Kappe 14 ist als nach distal gewölbte Halbkugel ausgebildet. Der Draht 16 ist koaxial in eine Sackbohrung eingelötet, die koaxial in die innere Stirnfläche des Stopfens 12 eingearbeitet ist.

In dem Ausführungsbeispiel der Figur 2 ist die Kappe 14 ebenfalls als Halbkugel ausgebildet. Durch die Kontaktspitze, d.h. durch den Stopfen 12 und die Kappe 14 durchgehend ist eine Durchtrittsbohrung 18, die achsparallel außermittig verläuft, vorgesehen. Die den Draht 16 aufnehmende Sackbohrung ist dementsprechend achsparallel und außermittig diametral zu der Durchtrittsbohrung 18 angeordnet.

In dem Ausführungsbeispiel der Figur 3 ist die Kappe 14 ebenfalls als Halbkugel ausgebildet. Die Durchtrittsbohrung 18 durchsetzt den Stopfen 12 und die Kappe 14 koaxial. Der Draht 16 ist in eine axial verlaufende in den Außenumfang des Stopfens 12 eingearbeitete Nut eingelötet.

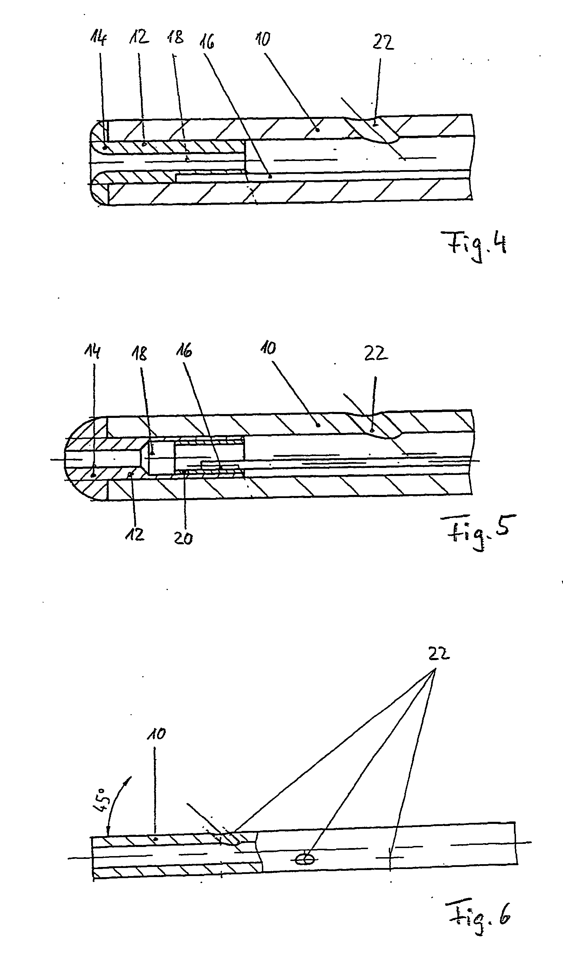

In dem Ausführungsbeispiel der Figur 4 ist die Kappe 14 als flacher Teller ausgebildet, der von einer ebenen distalen Stirnfläche ausgehend am Außenumfang mit einem Krümmungsradius gewölbt in die zylindrische Umfangsfläche des Katheters 10 übergeht. Wie in dem Ausführungsbeispiel der Figur 3 verläuft die Durchtrittsbohrung 18 koaxial und der Draht 16 ist in eine axiale Umfangsnut eingesetzt.

In dem Ausführungsbeispiel der Figur 5 ist die Kappe 14 als Halbkugel ausgebildet. Die Durchtrittsbohrung 18 verläuft koaxial. Gegen das innere Ende des Stopfens 12 hin ist die Durchtrittsbohrung 18 in ihrem Durchmesser erweitert. In diesen erweiterten Abschnitt der Durchtrittsbohrung 18 ist eine Hohlbuchse 20 eingesetzt, an deren Innenwandung der Draht 16 leitend befestigt ist.

Weist die Kontaktspitze eine axial durchgehende Durchtrittsbohrung 18 auf, so kann die durch den Katheter 10 zugeführte Flüssigkeit, z.B. ein Anästhetikum, durch diese Durchtrittsbohrung 18 austreten. Ist die Kontaktspitze geschlossen, wie z.B. beim Ausführungsbeispiel der Figur 1, so sind im distalen Endbereich des Katheters 10 hinter der Kontaktspitze eine oder mehrere Austrittsöffnungen 22 vorgesehen, durch welche die zugeführte Flüssigkeit austreten kann. Solche Austrittsöffnungen 22 können auch in den Fällen vorgesehen sein, in welchen die Kontaktspitze eine axiale Durchtrittsbohrung 18 aufweist, um den Querschnitt für den Austritt der zugeführten Flüssigkeit zu vergrößern.

Die Austrittsöffnungen 22 können eine beliebige Form, Querschnittsgröße und Anordnung aufweisen. Bevorzugt ist eine Ausführung, wie sie insbesondere in Figur 6 dargestellt ist. In dieser Ausführung sind die Austrittsöffnungen 22 so in die Wandung des Katheters 10 eingebracht, dass die Mittelachse der Austrittsöffnung 22 einen spitzen Winkel mit der Mittelachse des Katheters 10 einschließt, z.B. einen Winkel von 45°, wobei sich dieser spitze Winkel in distaler Richtung öffnet. Auf diese Weise tritt die zugeführte Flüssigkeit über die Austrittsöffnungen 22 nach distal gerichtet aus. Es sind drei Austrittsöffnungen 22 vorgesehen, die gegeneinander in Umfangsrichtung um jeweils 120° gegeneinander versetzt sind. Dadurch wird ein gleichmäßiges Austreten der zugeführten Flüssigkeit über den gesamten Umfang des distalen Endes des Katheters 10 gewährleistet. Damit die Wandung des Katheters 10 durch die Austrittsöffnungen 22 nicht zu stark geschwächt wird, sind die Austrittsöffnungen 22 vorzugsweise axial gegeneinander versetzt angeordnet.

## Patentansprüche

1. Katheter für die Nervenblockade in der Anästhesie, der aus einem flexiblen Kunststoffschlauch besteht, mit einem in dem Katheter (10) angeordneten Draht (16) für die Elektrostimulation, dessen proximales Ende am proximalen Ende des Katheters (10) elektrisch kontaktierbar ist und der zu einer distal aus dem Katheter (10) herausragenden Kontaktspitze führt, **dadurch gekennzeichnet, dass** die Kontaktspitze ein Metallteil ist, welches aus einem Stopfen (12) und einer Kappe (14) besteht, dass der Stopfen (12) koaxial in das distale Ende des Katheters (10) einsetzbar ist, dass die Kappe (14) vor dem distalen Ende des Katheters (10) angeordnet ist und dessen distale Randstirnfläche übergreift, dass der Querschnitt des Drahtes (16) kleiner ist als der freie Innenquerschnitt des Katheters (10) und dass der Draht (16) mit seinem distalen Ende elektrisch leitend mit dem Stopfen (12) verbunden ist.

2. Katheter nach Anspruch 1, **dadurch gekenzeichnet,** dass der Stopfen (12) und die Kappe (14) ein einstückiges Metallteil bilden.

3. Katheter nach Anspruch 1 oder 2, **dadurch gekennzeichnet, dass** der Draht (16) mit dem Stopfen (12) weichgelötet, geklebt, geprägt oder lasergeschweißt verbunden ist.

4. Katheter nach einem der Ansprüche 1 bis 3, **dadurch gekennzeichnet, dass** die Kappe (14) stumpf gewölbt ist und an ihrem Außenumfang stufenlos in die Äußenumfangkontur des Katheters (10) übergeht.

5. Katheter nach Anspruch 4, **dadurch gekennzeichnet, dass** die Kappe (14) halbkugelförmig ausgebildet ist.

6. Katheter nach einem der vorhergehenden Ansprüche, **dadurch gekennzeichnet , dass** eine Durchtrittsbohrung (18) axial durch den Stopfen (12) und die Kappe (14) hindurchführt.

7. Katheter nach einem der vorhergehenden Ansprüche, **dadurch gekennzeichnet , dass** die Wandung des Katheters (10) in dessen distalem Endbereich hinter dem Stopfen (12) wenigstens eine Austrittsöffnung (22) aufweist.

8. Katheter nach Anspruch 7, **dadurch gekennzeichnet, dass** die Mittelachse der wenigstens einen Austrittsöffnung (22) mit der Mittelachse des Katheters (10) einen sich in distaler Richtung öffnenden spitzen Winkel kleiner als 90° einschließt.

9. Katheter nach Anspruch 7 oder 8, **dadurch gekennzeichnet, dass** drei im Umfangswinkel um jeweils 120° versetzte Austrittsöffnungen (22) vorgesehen sind, die vorzugsweise axial gegeneinander versetzt sind.

## Claims

1. A catheter for a nerve block in anaesthesia, which consists of a flexible plastic tube, with a wire (16) disposed in the catheter (10) for electrical stimulation, the proximal end of which is electrically contactable at the proximal end of the catheter (10) and which leads to a contact tip protruding distally from the catheter (10),
**characterised in that** the contact tip is a metal part consisting of a plug (12) and a cap (14),
**in that** the plug (12) is can be inserted coaxially into the distal end of the catheter (10),
**in that** the cap (14) is disposed in front of the distal end of the catheter (10) and overlaps its distal edge surface,
**in that** the cross section of the wire (16) is smaller than the free internal cross section of the catheter (10),
**and in that** the wire (16) is connected in an electrically conductive manner by its distal end to the plug (12).

2. A catheter according to Claim 1,
**characterised in that** the plug (12) and the cap (14) form a single metal part.

3. A catheter according to Claim 1 or 2,
**characterised in that** the wire (16) is soft-soldered, glued, embossed or laser-welded to the plug (12).

4. A catheter according to one of Claims 1 to 3,
**characterised in that** the cap is (14) bluntly curved, and makes a smooth transition at its outer periphery into the outer peripheral contour of the catheter (10).

5. A catheter according to Claim 4,
**characterised in that** the cap (14) is designed in the shape of a hemisphere.

6. A catheter according to one of the preceding Claims,
**characterised in that** a through-hole (18) passes axially through the plug (12) and the cap (14).

7. A catheter according to one of the preceding Claims,
**characterised in that** the wall of the catheter (10 comprises at least one outlet opening (22) in its distal end region behind the plug (12).

8. A catheter according to Claim 7,
**characterised in that** the centre axis of the at least one outlet opening (22) encloses with the centre axis of the catheter (10) an acute angle of less than 90°, opening in the distal direction.

9. A catheter according to Claim 7 or 8,
**characterised in that** three outlet openings (22) offset by 120° in the circumferential angle are provided, and are preferably offset axially to each other.

## Revendications

1. Cathéter pour blocs de nerfs dans l'anesthésie composé d'un tuyau souple en plastique, doté d'un fil (16) disposé dans le cathéter (10) pour l'électrostimulation, dont l'extrémité proximale peut être mise en contact électrique sur l'extrémité proximale du cathéter et qui conduit à un contact de pointe faisant saillie du cathéter (10) de manière distale,
**caractérisé en ce que**
le contact de pointe est une partie métallique composée d'un tampon (12) et d'un capuchon (14), **en ce que** le tampon (12) peut être placé coaxialement dans l'extrémité distale du cathéter (10), **en ce que** le capuchon (14) est disposé avant l'extrémité distale du cathéter (10) et empiète sur sa surface frontale périphérique distale, **en ce que** la section transversale du fil (16) est inférieure à la section transversale intérieure libre du cathéter (10) et **en ce que** l'extrémité distale du fil (16) est reliée par conduction électrique au tampon (12).

2. Cathéter selon la revendication 1,
**caractérisé en ce que**
le tampon (12) et le capuchon (14) forment une partie métallique d'une seule pièce.

3. Cathéter selon la revendication 1 ou 2,
**caractérisé en ce que**
le fil (16) est relié au tampon (12) par brasage tendre, par collage, par gaufrage ou par soudage laser.

4. Cathéter selon l'une quelconque des revendications 1 à 3,
**caractérisé en ce que**
le capuchon (14) est bombé bord à bord et passe graduellement sur sa périphérie extérieure dans le contour de la périphérie extérieure du cathéter (10).

5. Cathéter selon la revendication 4,
**caractérisé en ce que**
le capuchon (14) est conçu sous forme de demi-sphère.

6. Cathéter selon l'une quelconque des revendications précédentes,
**caractérisé en ce qu'**
un alésage traversant (18) traverse axialement le tampon (12) et le capuchon (14).

7. Cathéter selon l'une quelconque des revendications précédentes,
**caractérisé en ce que**
la paroi du cathéter (10) présente au moins un orifice de sortie (22) dans sa zone d'extrémité distale derrière le tampon (12).

8. Cathéter selon la revendication 7,
**caractérisé en ce que**
l'axe médian de l'au moins un orifice de sortie (22) avec l'axe médian du cathéter (10) renferme au moins un angle aigu s'ouvrant dans la direction distale et inférieur à 90°.

9. Cathéter selon la revendication 7 ou 8,
**caractérisé en ce qu'**
on a prévu trois orifices de sortie (22) déportés de respectivement 120° dans l'angle périphérique et déportés de préférence axialement l'un contre l'autre.
